# EUROPÄISCHE PATENTSCHRIFT

(11) **EP 0 569 811 B1**
(45) Veröffentlichungstag und Bekanntmachung des Hinweises auf die Patenterteilung: **17.09.1997**
(21) Anmeldenummer: 93107140.1
(22) Anmeldetag: 03.05.1993
(51) Int. Cl.: C07K 5/03, A61K 38/55, A61K 31/385, C07D 409/12, C07D 409/14

(54) **Dithiolanylglycinhaltige HIV-Proteaseinhibitoren vom Hydroxyethylenisostertyp**
Dithiolanylglycine containing HIV-protease inhibitors of the hydroxyethylene isostere type
Inhibiteurs de la HIV-protéase du type isostère hydroxyéthylène contenant dithiolanylglycine

(30) Priorität: 14.05.1992 DE 4215874
(43) Veröffentlichungstag der Anmeldung: 18.11.1993
(73) Patentinhaber: BAYER AG, 51368 Leverkusen (DE)
(72) Erfinder: Häbich, Dieter, Dr., W-5600 Wuppertal 1 (DE); Bender, Wolfgang, Dr., W-5600 Wuppertal 1 (DE); Hansen, Jutta, Dr., W-5600 Wuppertal 1 (DE); Paessens, Arnold, Dr., W-5657 Haan (DE)

(56) Entgegenhaltungen:
- EP-A- 0 357 332
- EP-A- 0 403 828
- EP-A- 0 437 729
- WO-A-92/10509
- WO-A-92/10510
- JOURNAL OF MEDICINAL CHEMISTRY Bd. 34, Nr. 8, 1991, Washington, US, Seiten 2305-2314, J.R. HUFF

## Beschreibung

Die Erfindung betrifft Dithiolanylglycinhaltige HIV-Proteaseinhibitoren vom Hydroxyethylenisostertyp, Verfahren zu ihrer Herstellung und ihre Verwendung als retrovirale Mittel.

Es wurde bereits versucht, Peptide und Pseudopeptide, die teilweise auch renininhibitorisch wirksam sind, bei der Bekämpfung von AIDS einzusetzen [vgl. WO 91/ 06561; WO 90/09191; WO 90/12804; EP 393 445; EP 402 646; EP 373 576; WO 91/06 501; EP 459 465 und EP 437 729].

Die vorliegende Erfindung betrifft Dithiolanylglycinhaltige HIV-Proteaseinhibitoren vom Hydroxyethylenisostertyp der allgemeinen Formel (I) 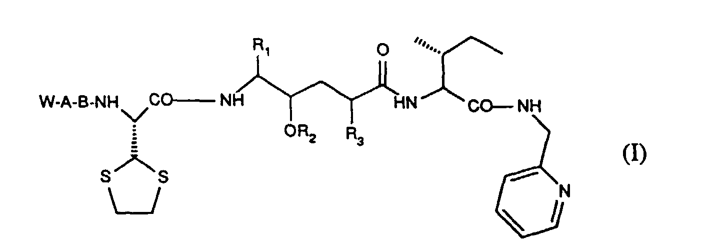 in welcher
- W: für Wasserstoff oder für eine typische Aminoschutzgruppe steht, oder für eine Gruppe der Formel R⁴-CO- steht,
worin
- R⁴: Chinolyl, Indolyl, Pyridyl, Morpholino oder Piperazinyl bedeutet, oder
einen Rest der Formel 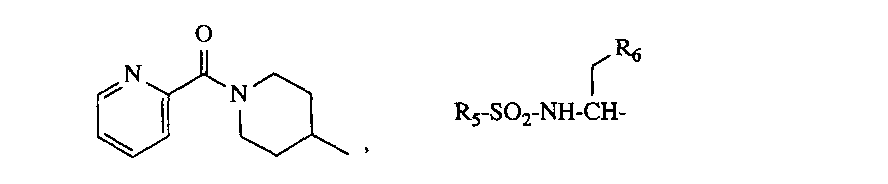 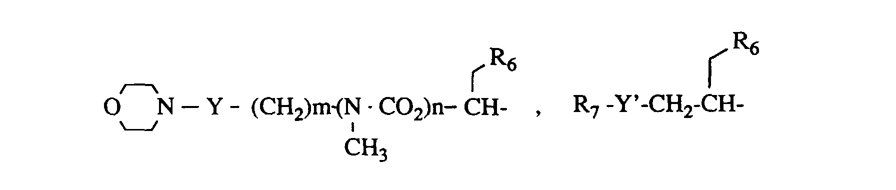 oder 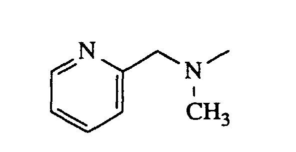 bedeutet,
worin
R⁵ geradkettiges oder verzweigtes Alkyl mit bis zu 14 Kohlenstoffatomen bedeutet, das gegebenenfalls durch Phenyl oder Naphthyl substituiert ist, oder Benzyloxy oder Aryl mit 6 bis 10 Kohlenstoffatomen bedeutet, welches seinerseits durch Alkyl mit bis zu 4 Kohlenstoffatomen substituiert ist, oder über N-gebundenes Morpholino oder Pyrrolidinyl bedeutet,
R⁶ Phenyl oder Naphthyl bedeutet,
R⁷ die oben angegebene Bedeutung von R⁵ hat aber nicht für über N-gebundenes Morpholino oder Pyrrolidinyl steht,
Y und Y' unabhängig voneinander die CO- oder SO₂-Gruppe bedeuten,
m eine Zahl 0, 1 oder 2 bedeutet,
n eine Zahl 0 oder 1 bedeutet,
- A und B: gleich oder verschieden sind und für eine direkte Bindung oder für einen Rest der Formel 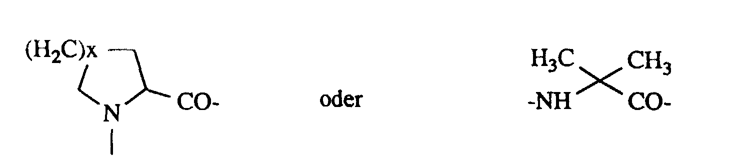 stehen , worin
- x: die Zahl 1 oder 2 bedeutet
oder
für eine Gruppe der Formel 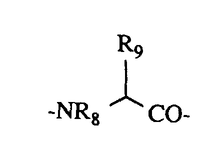 stehen
worin
- R⁸: Wasserstoff oder geradkettiges oder verzweigtes Alkyl mit bis zu 4 Kohlenstoffatomen bedeutet,
- R⁹: Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel -NR¹⁰R¹¹ oder R¹²-OC- substituiert ist,
worin
R¹⁰ und R¹¹ unabhängig voneinander für Wasserstoff, geradkettiges oder verzweigtes Alkyl mit bis zu 8 Kohlenstoffatomen oder Phenyl stehen,
und
R¹² Hydroxy, Benzyloxy, Alkoxy mit bis zu 6 Kohlenstoffatomen oder die oben aufgeführte Gruppe -NR¹⁰R¹¹ bedeutet, oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR¹⁰R¹¹ substituiert ist,
worin
R¹⁰ und R¹¹ die oben angegebene Bedeutung haben, oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
R¹ für geradkettiges oder verzweigtes Alkyl mit bis zu 3 Kohlenstoffatomen steht, das durch Cyclohexyl oder Phenyl substituiert ist,
R² für Wasserstoff oder für geradkettiges oder verzweigtes Alkyl mit bis zu 4 Kohlenstoffatomen steht, oder für eine Hydroxyschutzgruppe steht,
R³ für geradkettiges oder verzweigtes Alkyl oder Alkenyl mit jeweils bis zu 6 Kohlenstoffatomen oder für Benzyl steht,
und deren physiologisch unbedenklichen Salze.

Die erfindungsgemäßen Verbindungen der allgemeinen Formel (I), haben mehrere asymmetrische Kohlenstoffatome. Sie können unabhängig voneinander in der D- oder L-Form vorliegen. Die Erfindung umfaßt die optischen Antipoden ebenso wie die Isomerengemische oder Racemate. Bevorzugt liegen die Gruppen A, B und D unabhängig voneinander in der optisch reinen, bevorzugt in der L-Form vor.

Der Rest der allgemeinen Formel (II) 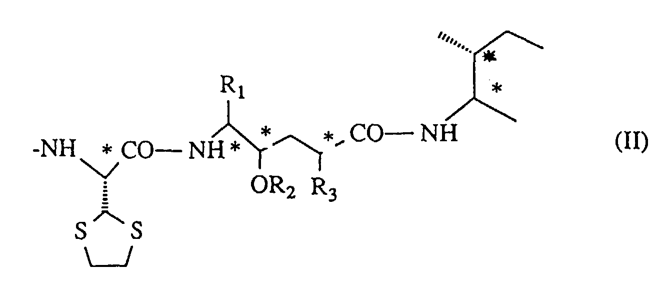 besitzt 6 asymmetrische Kohlenstoffatome (*). Sie können unabhängig voneinander in der R- oder S-Konfiguration vorliegen.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 9-Fluorenylmethyloxycarbonyl, Methoxycarbonyl, tert.Butoxycarbonyl, Allyloxycarbonyl, Vinyloxycarbonyl, 2-Nitrobenzyloxycarbonyl, Formyl, Acetyl, 2,2,2-Trifluoracetyl, 2,2,2-Trichloracetyl, Benzoyl oder Pyridylmethoxycarbonyl.

Die erfindungsgemäßen Verbindungen der allgemeinen Formel (I) können in Form ihrer Salze vorliegen. Dies können Salze mit anorganischen oder organischen Säuren oder Basen sein.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyldimethylsilyl, tert.Butyldiphenylsilyl, Triphenylsilyl, Trimethylsilylethoxycarbonyl, Benzyl, Triphenylmethyl (Trityl), Monomethoxytrityl (MMTr), Dimethoxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert-Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzyl und tert.Butyldimethylsilyl.

Bevorzugt sind Verbindungen der allgemeinen Formel (I),
in welcher
- W: für Wasserstoff, tert.Butyloxycarbonyl (BOC), 9-Fluorenylmethyloxycarbonyl (Fmoc), Benzyloxycarbonyl (Z) oder Pyridylmethoxycarbonyl steht, oder für eine Gruppe der Formel R⁴-CO- steht
worin
R⁴ Chinolyl, Indolyl, Pyridyl, Morpholino oder Piperazinyl bedeutet, oder
einen Rest der Formel 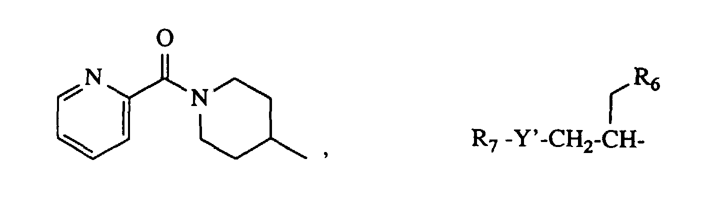 oder 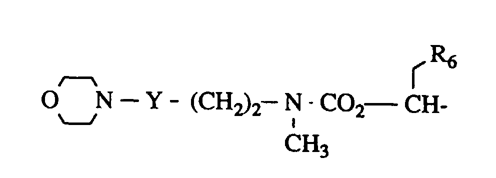 bedeutet,
worin
Y und Y' unabhängig voneinander die CO- oder SO₂-Gruppe bedeuten,
R⁶ Phenyl oder Naphthyl bedeutet,
R⁷ geradkettiges oder verzweigtes Alkyl mit bis zu 8 Kohlenstoffatomen, Tolyl, Benzyloxy, Phenyl oder Naphthyl bedeutet,
- A und B: unabhängig voneinander
für eine direkte Bindung oder
für Prolin, Alanin, Arginin, Asparaginsäure, Asparagin, Glutaminsäure, Glutamin, Histidin, Leucin, Methionin, Threonin, Tyrosin, Cystein, Glycin, Isoleucin, Lysin, Phenylalanin, Serin, Tryptophan oder Valin stehen,
- R¹: für Benzyl oder für Methylcyclohexyl steht,
- R²: für Wasserstoff steht,
- R³: für geradkettiges oder verzweigtes Alkyl oder Alkenyl mit jeweils bis zu 4 Kohlenstoffatomen oder Benzyl steht,
und deren physiologisch unbedenklichen Salze.

Besonders bevorzugt sind Verbindungen der allgemeinen Formel (I),
in welcher
- W: für Wasserstoff oder tert.Butyloxycarbonyl (BOC) steht, oder für eine Gruppe der Formel R⁴-CO- steht,
worin
- R⁴: Chinolyl oder Indolyl bedeutet, oder
einen Rest der Formel 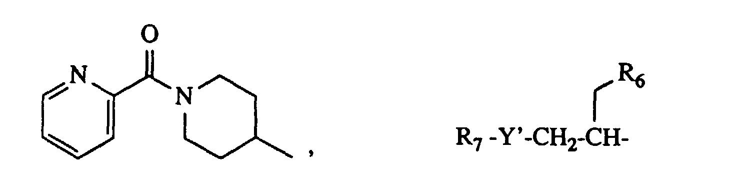 oder 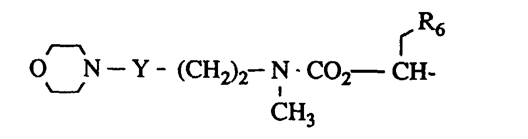 bedeutet,
worin
R⁶ Phenyl oder Naphthyl bedeutet
R⁷ geradkettiges oder verzweigtes Alkyl mit bis zu 4 Kohlenstoffatomen, Tolyl, Benzyloxy, Phenyl oder Naphthyl bedeutet,
- Y und Y': unabhängig voneinander die -CO- oder SO₂-Gruppe bedeuten,
- A und B: unabhängig voneinander für eine direkte Bindung, oder für Phenylalanin stehen,
- R¹: für Benzyl oder Methylcyclohexyl steht,
- R²: für Wasserstoff steht,
- R³: für geradkettiges oder verzweigtes Alkyl mit bis zu 3 Kohlenstoffatomen, Allyl oder Benzyl steht
und deren physiologisch unbedenklichen Salze.

Außerdem wurden Verfahren zur Herstellung der erfindungsgemäßen Verbindungen der allgemeinen Formel (I) gefunden, dadurch gekennzeichnet, daß man

### [A] im Fall, daß A und B für eine direkte Bindung stehen,

Verbindungen der allgemeinen Formel (III) 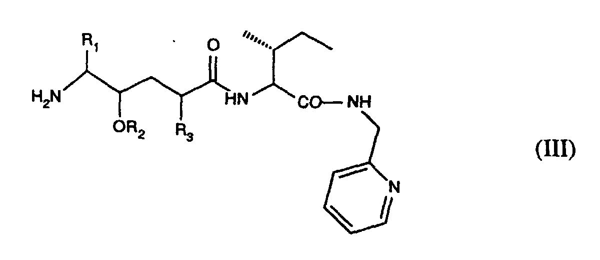 in welcher
R¹, R² und R³ die oben angegebene Bedeutung haben,
zunächst mit Verbindungen der allgemeinen Formel (IV) 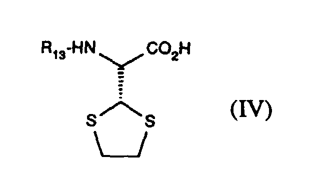 in welcher
- R¹³: für eine der oben aufgeführten Aminoschutzgruppen, vorzugsweise für Boc steht,
in inerten organischen Lösemitteln, in Anwesenheit einer Base und eines Hilfsstoffes umsetzt,
anschließend die Schutzgruppe R¹³ nach denen in der Peptidchemie üblichen Methoden abspaltet (W = H) und im Fall, daß W ≠ H, in einem letzten Schritt mit Verbindungen der allgemeinen Formel (V)

W'-X (V)

in welcher
W' die oben angegebene Bedeutung von W hat, aber nicht für Wasserstoff steht,
und
X in Abhängigkeit von der jeweiligen Bedeutung der unter W oben aufgeführten Substituenten für Hydroxy oder Halogen steht,
ebenfalls in organischen Lösemitteln und in Anwesenheit einer Base und eines Hilfsstoffes umsetzt
und

### [B] im Fall, daß A und/oder B nicht für eine direkte Bindung stehen,

Verbindungen der allgemeinen Formel (III) entweder direkt mit Verbindungen der allgemeinen Formel (VI) 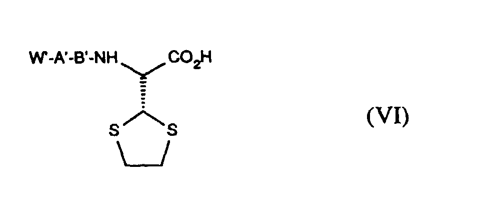 in welcher
W' die oben angegebene Bedeutung hat
und
A' und B' die oben angegebene Bedeutung von A und B haben aber nicht gleichzeitig für eine Bindung stehen,
oder sukzessive zunächst entweder mit Verbindungen der allgemeinen Formel (VII) 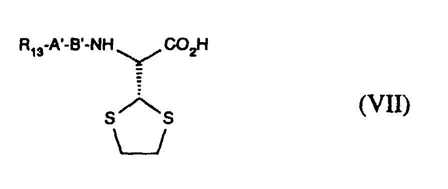 in welcher
R¹³, A' und B' die oben angegebene Bedeutung haben,
in inerten Lösemitteln und in Anwesenheit einer Base und eines Hilfsstoffes umsetzt,
anschließend unter vorgeschalteter Abspaltung der Schutzgruppe R¹³ wie unter [A] beschrieben, mit Verbindungen der allgemeinen Formel (V) umsetzt,
oder

Verbindungen der allgemeinen Formel (III) zunächst mit Verbindungen der allgemeinen Formel (IV) wie unter [A] beschrieben umsetzt, die Schutzgruppe R¹³ abspaltet und in einem letzten Schritt mit Verbindungen der allgemeinen Formel (VIII)

W'-A'-B'-OH (VIII)

in welcher
W', A' und B' die oben angegebene Bedeutung haben,
nach denen in der Peptidchemie üblichen Methoden, gegebenenfalls unter Aktivierung der Carbonsäurefunktion und unter einer vorgeschalteten Deblockierung der Aminfunktion, in inerten organischen Lösemitteln und in Anwesenheit einer Base und eines Hilfststoffes umsetzt,
und im Fall, daß W für Wasserstoff steht, wie unter [A] beschrieben, die Schutzgruppe abspaltet,
und gegebenenfalls eine Trennung der Diastereomere durchführt.

Die erfindungsgemäßen Verfahren sollen beispielhaft durch das Verfahren [A] in dem folgenden Formelschema erläutert werden:

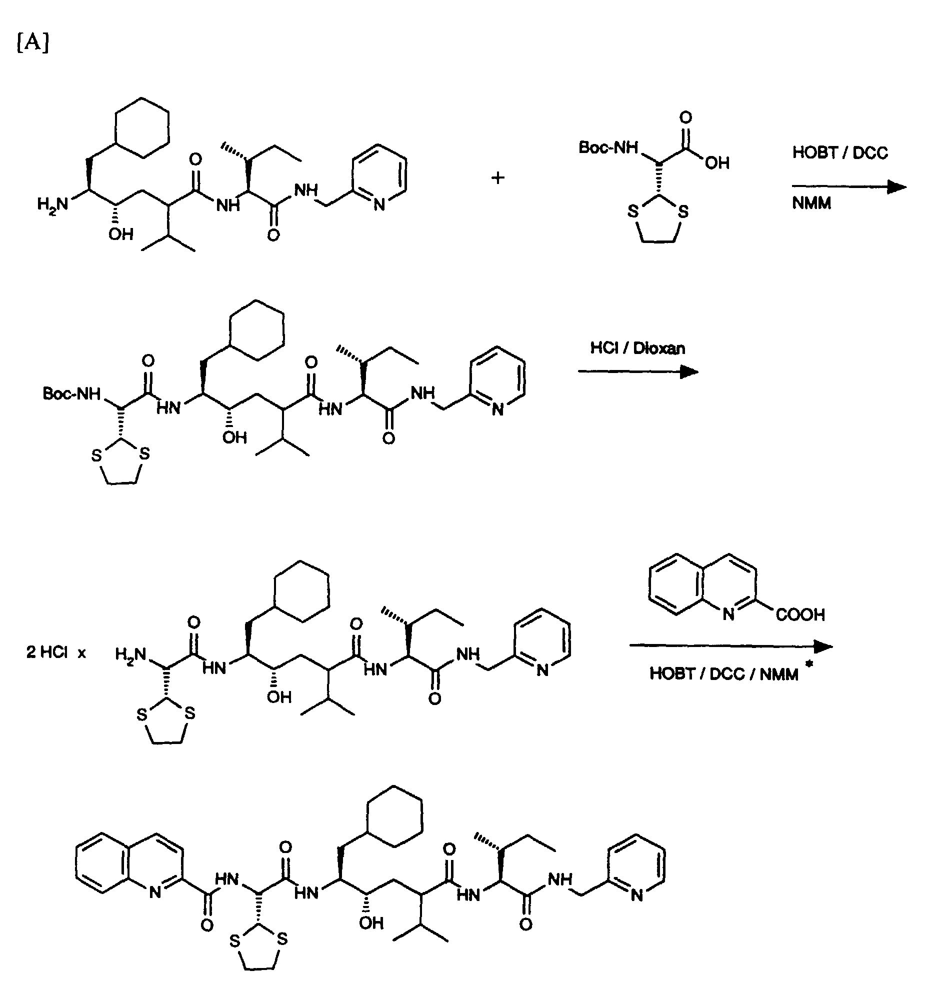

Als Lösemittel eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid oder n-Propanol.

Als Hilfsstoffe für die jeweiligen Peptidkupplungen werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tri(dimethylamino)phosphonium-hexafluorophosphat, oder 1-Hydroxybenzotriazol und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt sind Dicyclohexylcarbodiimid, N-Methylmorpholin und 1-Hydroxybenztriazol.

Die Abspaltung der Aminoschutzgruppe erfolgt in an sich bekannter Weise unter sauren oder basischen Bedingungen, oder reduktiv durch katalystische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Umsetzungen werden im allgemeinen in einem Temperaturbereich von -20°C bis +80°C, bevorzugt von 0°C bis +60°C durchgeführt.

Im allgemeinen wird die Umsetzung bei Normaldruck durchgeführt Es ist aber auch möglich, bei Unterdruck oder bei Überdruck zu arbeiten (z.B. von 0,5 bis 5 bar).

Die Verbindungen der allgemeinen Formel (VIII) sind an sich bekannt und können durch Umsetzung eines entsprechenden Bruckstückes, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, gegebenenfalls in aktivierter Form, und durch Wiederholung dieses Vorgangs mit entsprechenden Bruchstücken hergestellt werden [vgl. hierzu EP 300 189 und H. Bühlmayer et al., J. Med. Chem. 31, 1839 (1988)]; anschließend können gegebenenfalls Schutzgruppen abgespalten oder gegen andere Schutzgruppen ausgetauscht werden [vgl. Houben-Weyl, Methoden der organischen Chemie, Synthese von Peptiden II, 4. Aufl. Bd. 15/1, 15/2, Georg Thieme Verlag, Stuttgart].

Als Hilfsstoffe für die Einführung des Restes W' (V)/(VIII) werden die oben unter der Peptidkupplung aufgeführten Kondensationsmittel eingesetzt.

Die Abspaltung der Aminoschutzgruppen kann ebenfalls nach üblicher Methode mit Säuren, wie beispielsweise Chlorwasserstoffsäure oder Trifluoressigsäure erfolgen.

Die Verbindungen der allgemeinen Formel (III) sind an sich bekannt und können in Analogie zu denen in der EP 437 729 und EP 432 595 publizierten Methoden hergestellt werden [vgl. hierzu außerdem D. J. Plata et al., THL 32, 3623 (1991) und J.P. Vacca et al., J. Med. Chem. 34, 1228 (1991)].

Die Verbindungen der allgemeinen Formeln (IV), (VI) und (VII) sind teilweise bekannt oder neu und können hergestellt werden indem man Dithiolanylglycin der allgemeinen Formel (IX) 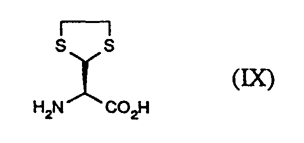 gegebenenfalls unter Aktivierung mit Reagenzien, wie beispielsweise (R¹³O-CO)₂O, R¹³-O-CO-Cl oder (R¹³-O-CO)O-N-succinimid, die Aminoschutzgruppe R¹³ (siehe Formel IV) in einem der oben aufgeführten Lösemittel / Lösemittelgemischen, vorzugsweise Dioxan / H₂O mit Natriumhydroxid oder in Dioxan mit Triethylamin einführt, und im Fall der Verbindungen der allgemeinen Formeln (VI) und (VII) zunächst die Schutzgruppe R¹³, wie oben beschrieben abspaltet und im Sinne einer wie oben beschriebenen Peptidknüpfung die Reste W'-A'-B-OH bzw. R¹³-A'-B'-OH, worin W', A', B' und R¹³ die oben angegebene Bedeutung haben, ebenfalls wie oben beschrieben, einführt.

Die Umsetzungen verlaufen in einem Temperaturbereich von 0° bis +50°C, bevorzugt bei Raumtemperatur und Normaldruck.

Die Verbindung der allgemeinen Formel (IX) ist (als Gemisch) bekannt [vgl. M.P. Mertes, A.A. Ramsey, J. Med. Chem. 12, 342(1969)].

Die Verbindungen der allgemeinen Formel (V) sind bekannt.

Die hier beschriebenen Inhibitoren sind Inhibitoren der HIV-Protease und sind als solche für alle Zwecke einsetzbar, für die Enzyminhibitoren geeignet sind. Dies ist zum Beispiel der Einsatz in der Diagnostik, um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen und der Spezifität enzymatischer Reaktionen verwenden.

Darüber hinaus wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.
Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert Die angegebenen IC₅₀-Werte beziehen sich auf die Substanzkonzentration, die unter den oben aufgeführten Testbedingungen eine 50%ige Hemmung der Protease-Aktivität bewirkt.

### Enzym assay, HIV-1

**Tabelle 1**

| Beispiel Nr. | IC₅₀(RP-HPLC)(M) HIV-1 |
|---|---|
| 1 | 1,4 x 10⁻⁹ |
| 2 | 1,3 x 10⁻⁹ |
| 3 | 1,3 x 10⁻⁸ |
| 4 | 1,6 x 10⁻⁸ |
| 5 | 1,4 x 10⁻⁹ |
| 8 | 1,2 x 10⁻⁹ |
| 9 | 1,0 x 10⁻⁹ |
| 10 | 6,8 x 10⁻⁸ |
| 11 | 1,4 x 10⁻¹⁰ |
| 12 | 9,8 x 10⁻⁸ |
| 13 | 6,4 x 10⁻¹¹ |
| 14 | 1,3 x 10⁻¹⁰ |
| 15 | 6,8 x 10⁻⁹ |
| 16 | 2,1 x 10⁻⁸ |
| 18 | 1,9 x 10⁻⁹ |
| 19 | 2,3 x 10⁻⁸ |
| 20 | 1,6 x 10⁻⁸ |
| 21 | 8,7 x 10⁻⁹ |

Außerdem zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phytohaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, währen die unbehandelte Zellkontrolle keine Syncytien aufwies.

Die IC₅₀-Werte wurden als die Konzentrationen der behandelten und infizierten Zellen ermittelt, bei der 50% (ca 10 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

**Tabelle II**

| Bsp.-Nr. | IC₅₀(µM) [PBL] | [H-9] |
|---|---|---|
| 1 | >1 | |
| 2 | 10 | |
| 3 | 1 | |
| 4 | 1 | |
| 5 | 0,4 | |
| 8 | 1 | |
| 9 | 0,04 | 0,011 |
| 10 | 5 | |
| 11 | 1 | |
| 12 | 5 | |
| 13 | 0,05 | 0,038 |
| 14 | >1 | |
| 15 | 10 | |
| 16 | 10 | |
| 18 | 0,4 | |
| 19 | <0,08 | |
| 20 | <0,4 | |
| 21 | <0,4 | |

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneintittels sowie dem Zeitraum bzw. Intervall, innerhalb welchem die Verabreichung erfolgt.

### Anhang zum experimentellen Teil

### I. Liste der verwendeten Laufmittelgemische zur Chromatographie:

I Dichlormethan Methanol
II Toluol : Ethylacetat
III Acetonitril : Wasser
IV Dichlormethan : Tetrahydrofuran
V Toluol: Acetonitril

### II. Aminosäuren

Im allgemeinen erfolgt die Bezeichnung der Konfiguration durch das Vorausstellen eines L bzw. D vor der Aminosäureabkürzung, im Fall des Racemats durch ein D,L-, wobei zur Vereinfachung bei L-Aminosäuren die Konfigurationsbezeichnung unterbleiben kann und dann nur im Fall der D-Form bzw. des D,L-Gemisches eine explizierte Bezeichnung erfolgt.
- Ala: L-Alanin
- Arg: L-Arginin
- Asn: L-Asparagin
- Asp: L-Asparaginsäure
- Cys: L-Cystein
- Gln: L-Glutamin
- Glu: L-Glutaminsäure
- Gly: L-Glycin
- His: L-Histidin
- Ile: L-Isoleucin
- Leu: L-Leucin
- Lys: L-Lysin
- Met: L-Methionin
- Pro: L-Prolin
- Phe: L-Phenylalanin
- Ser: L-Serin
- Thr: L-Threonin
- Trp: L-Tryptophan
- Tyr: L-Tyrosin
- Val: L-Valin

### III. Abkürzungen

- Z: Benzyloxycarbonyl
- Boc: tert.Butyloxycarbonyl
- CMCT: 1-Cyclohexyl-3-(2-morpholino-ethyl)-carbodiimidmetho-p-toluolsulfonat
- DCC: Dicyclohexylcarbodiimid
- DMF: Dimethylformamid
- HOBT: 1-Hydroxybenzotriazol
- Ph: Phenyl
- THF: Tetrahydrofuran
- Cha: Cyclohexylalanin
- Aib: 2-Amino-2-methylpropionsäure
- NMM: N-Methylmorpholin

### Herstellungsbeispiele

### Beispiel 1

1-{(2R,S,4S,5S)-5-[(2R)-N-(tert.Butoxycarbonyl)-2-amino-2-[2-(1,3-dithiolan-2-yl)]ethanoyl]amino-6-cyclohexyl-4-hydroxy-2-(1-methyl)ethyl-hexanoyl)-S-isoleucinyl-2-pyridylmethylamid 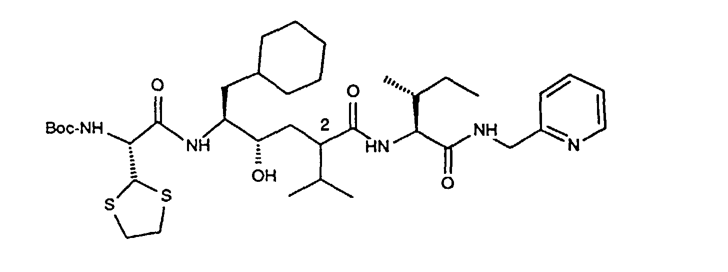 Eine auf 0°C gekühlte, gerührte Lösung von 614 mg (2,20 mmol) (2R)-N-(tert.Butoxycarbonyl)-2-amino-2-[2-(1,3-dithiolan-2-yl)]essigsäure (EP 412 350) und 337 mg (2,20 mmol) HOBT in 10 ml wasserfreiem Dichlormethan wird mit 434 mg (2,10 mmol) DCC versetzt und 5 min gerührt. Danach wird die Lösung von 1,10 g (2,20 mmol) 1-{(2R, S, 4S, 5S)-[5-Amino-6-cyclohexyl-4-hydroxy-2-(1-methyl)-ethyl-hexanoyl]}-S-isoleucinyl-2-pyridylmethylamid Dihydrochlorid [EP 437 729] und 0,88 ml (8,0 mmol) N-Methylmorpholin in 10 ml Dichlormethan zugetropft. Das Kühlbad wird entfernt und die Reaktionsmischung darf 2 h bei Raumtemperatur rühren. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 90 g Kieselgel (Dichlormethan : Methanol 95:5). Man erhält 1,29 g (88% der Theorie) der Titelverbindung als helles Pulver.
Schmp.: 252°C (Zers.)
R_{f}= 0,19, 0,23, I (9:1)
MS (FAB): m/z = 736 (M+H)⁺

### Beispiel 2

1 - {(2R,S;4S,5S)-5-[(2R)-2-Amino-2-[2-(1,3-dithiolan-2-yl)]ethanoyl]-amino-6-cyclohexyl-4-hydroxy-2-(1-methyl)ethyl-hexanoyl}-S-isoleucinyl-2-pyridylmethylamid Dihydrochlorid 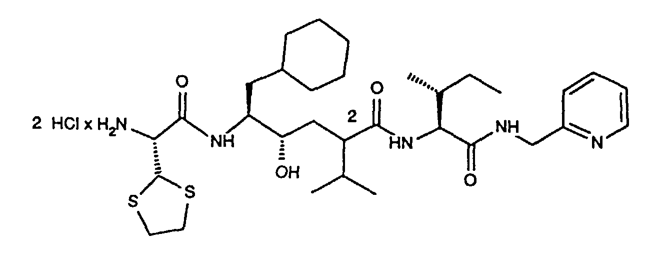 Eine Lösung von 2,41 g (3,28 mmol) der Verbindung aus Beispiel 1 in 17 ml einer 4 N Lösung gasförmigen Chlorwasserstoffs in wasserfreiem Dioxan wird 30 min bei 0°C gerührt. Danach gibt man 15 ml Toluol zu und engt im Vakuum ein. Dieser Vorgang wird noch zweimal wiederholt, dann wird der Rückstand mit Ether verrieben, abgesaugt und im Hochvakuum über KOH getrocknet. Man erhält 2,29 g (98% der Theorie) der Titelverbindung als farbloses Pulver.
Schmp.: ab 192°C (Zers.)
R_{f}=0,47 III (9:1)
MS (FAB): m/z = 636 (M+H)⁺

### Beispiel 3 und Beispiel 4

1-{(2R,4S,5S)-5-[(2R)-N-(tert.Butoxycarbonyl)-2-amino-2-[2-(1,3-dithiolan-2-yl)]-ethanoyl] amino-6-cyclohexyl-4-hydroxy-2-(phenyl)methyl-hexanoyl}-S-isoleucinyl-2-pyridylmethylamid 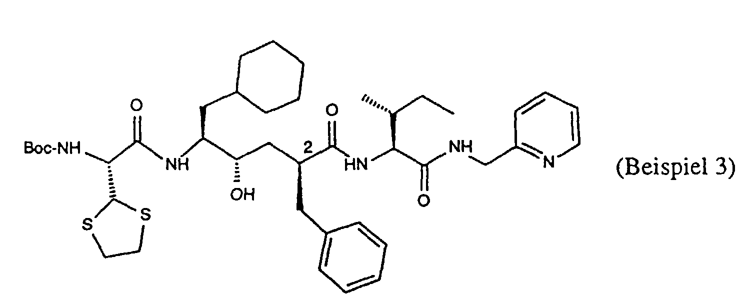 1-{(2S,4S,5S)-5[(2R)-N-(tert.Butoxycarbonyl)-2-amino-2-[2-(1,3-dithiolan-2-yl)]-ethanoyl]amino-6-cyclohexyl-4-hydroxy-2-(phenyl)methyl-hexanoyl}-S-isoleucinyl-2-pyridylmethylamid 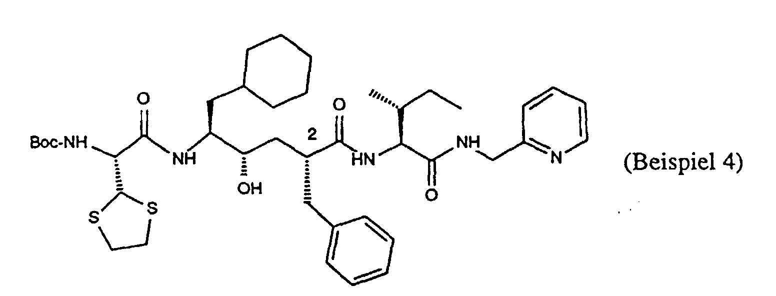 Wie für Beispiel 1 beschrieben erhält man aus 258 mg (0,92 mmol) (2R)-N-(tert.Butoxycarbonyl)-2-amino-2-[2-(1,3-dithiolan-2-yl)]essigsäure [EP 412 350] und 500 mg (0,84 mmol) 1-{(2R,S,4S,5S)-[5-Amino-6-cyclohexyl-4-hydroxy-2-(phenyl)-methyl-hexanoyl]-S-isoleucinyl-2-pyridylmethylamid Dihydrochlorid (hergestellt nach EP 437 729] und durch Chromatographie des Rohprodukts an 20 g Kieselgel (Laufmittelgemisch I, 95:5) 593 mg (90%) der Titelverbindung als Gemisch der 2(R,S)-Diastereomere. Durch chromatographische Auftrennung an 80 g Kieselgel erhält man 217 mg (33%) des unpolaren (2R)-Isomers als amorphes Pulver (Beispiel 3).
Beispiel 3: R_{f} = 0,65, I (9:1)
Beispiel 3: MS (FAB): m/z = 784 (M+H)⁺
Beispiel 4: 257 mg (39%) als Kristalle
Beispiel 4: Schmp. 201°C
Beispiel 4: R_{f}= 0,51, I (9:1)
Beispiel 4: MS (FAB): m/z = 784 (M+H)⁺

### Beispiel 5

1-{(2R,5,4S,5S)-5-[(2R)-N-(tert.Butoxycarbonyl)-2-amino-2-[2-(1,3-dithiolan-2-yl)]ethanoyl]amino-6-cyclohexyl-4-hydroxy-2-(2-propenyl)-hexanoyl}-S-isoleucinyl-2-pyridylmethylamid 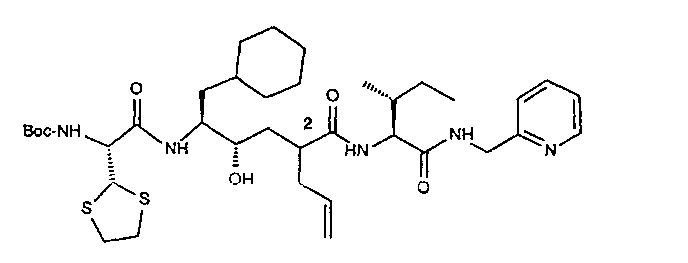 Wie für Beispiel 1 beschrieben erhält man aus 249 mg (0,89 mmol) (2R)-N-(tert.Butoxycarbonyl)-2-amino-2-[2-(1,3-dithiolan-2-yl)]essigsäure [EP 412 350] und 440 mg (0,81 mmol) 1-{(2R,S,4S,5S)-[5-Amino-6-cyclohexyl-4-hydroxy-2-(2-propenyl)-hexanoyl]}-S-isoleucinyl-2-pyridylmethylamid Dihydrochlorid [hergestellt nach EP 437 729] und durch Chromatographie des Rohprodukts an 24 g Kieselgel (Laufmittelgemisch I, 9:1) 553 mg (93%) der Titelverbindung als helles Pulver (Gemisch der 2 R,S-Diasteromere).
R_{f} = 0,48, 0,42 (I, 9:1)
MS (FAB): m/z = 734 (M+H)⁺

### Beispiel 6

1-{(2R,5,4S,5S)-5-[(2R)-2-Amino-2-[2-(1,3-dithiolan-2-yl)]ethanoyl]amino-6-cyclohexyl-4-hydroxy-2-(phenyl)methyl-hexanoyl}-S-isoleucinyl-2-pyridylmethylamid Dihydrochlorid 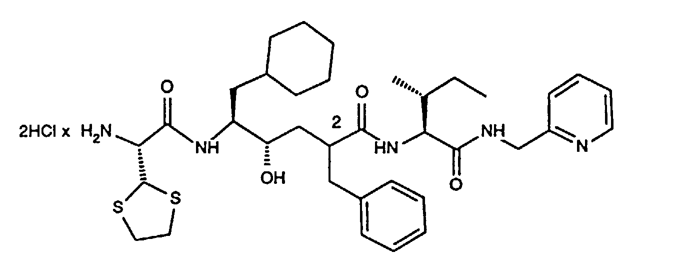 Wie für Beispiel 2 beschrieben erhält man aus 589 mg (0,75 mmol) der Verbindung aus Beispiel 3/4 (Gemisch der 2(R,S)-Diastereomere) 484 mg (86%) der Titelverbindung als farbloses Pulver.
R_{f} = 0,29, 0,34, (I, 9:1)
MS (FAB): m/z = 684 (M+H)⁺

### Beispiel 7

1-{(2R,S,4S,5S)-5-[(2R)-2-Amino-2-[2-(1,3-dithiolan-2-yl)]ethanoyl]-amino-6-cyclohexyl-4-hydroxy-2-(2-propenyl)-hexanoyl}-S-isoleucinyl-2-pyridylmethylamid 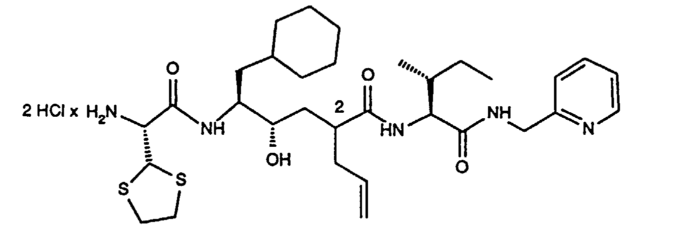 Wie für Beispiel 2 beschrieben erhält man aus 560 mg (0,91 mmol) der Verbindung aus Beispiel 5 452 mg (91%) der Titelverbindung als farbloses Pulver.
R_{f} = 0,08, III (9:1)
MS (FAB): m/z = 473 (M+H)⁺

### Beispiel 8 und Beispiel 9

1-{(2R,4S,5S)-5-[(2S)-3-(tert.Butylsulfonyl)-2-(1-naphthylmethyl)propanoyl]-(2R)-N-(tert.butoxycarbonyl)-2-amino-2-[2-(1,3-dithiolan-2-yl)]ethanoyl]-amino-6-cyclohexyl-4-hydroxy-2-(1-methyl)ethyl-hexanoyl}-S-isoleucinyl-2-pyridylmethylamid 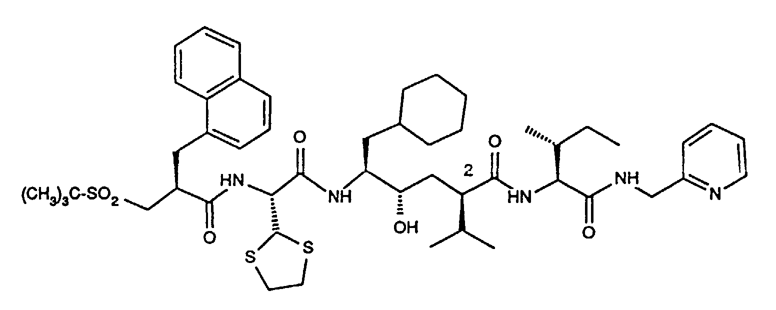 1-{(2S,4S,5S)-5-[(2S)-3-(tert.Butylsulfonyl)-2-(1-naphthylmethyl)propanoyl]-(2R)-N-(tert.butoxycarbonyl)-2-amino-2-[2-(1,3-dithiolan-2-yl)]ethanoyl]-amino-6-cyclohexyl-4-hydroxy-2-(1-methyl)ethyl-hexanoyl}-S-isoleucinyl-2-pyridylmethylamid 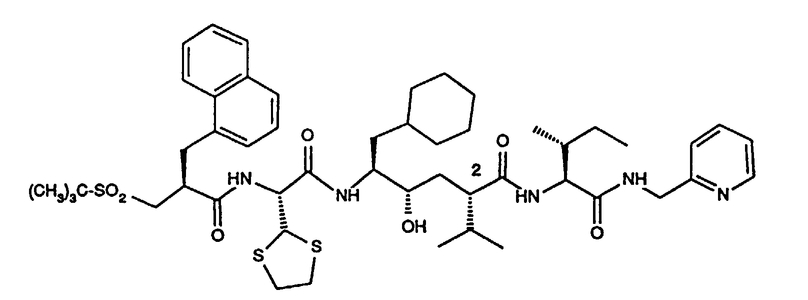 Eine auf 0°C gekühlte, gerührte Lösung von 0,80 g (2,40 mmol) (2S)-3-tert.Butylsulfonyl-2-(1-naphthylmethyl)-propionsäure [hergestellt nach H. Bühlmayer et al., J. Med. Chem. 31, 1839 (1988)] und 0,40 g (2,64 mmol) HOBT in 20 ml wasserfreiem Dichlormethan wird mit 0,52 g (2,52 mmol) DCC versetzt und 5 min gerührt. Danach wird eine Lösung von 1,55 g (2,19 mmol) der Verbindung aus Beispiel 2 und 0,96 ml (8,74 mmol) N-Methylmorpholin in 30 ml Dichlormethan zugetropft und die Reaktion darf 2 h bei Raumtemperatur rühren. Man trennt den entstandenen Harnstoff durch Filtration ab, engt das Filtrat im Vakuum ein und reinigt das Rohprodukt durch Chromatographie an 360 g Kieselgel (Dichlormethan : Methanol 95:5). Man erhält 586 mg (28% der Theorie) des unpolaren (2R)-Isomers als farbloses Pulver (Beispiel 8)
Schmp.: ab 209°C (Zers.)
R_{f}=0,20, I (95:5)
MS (FAB): m/z = 952 (M+H)⁺
und 690 mg (33%) des polaren (2S)-Isomers als farbloses Pulver (Beispiel 9) Schmp.: 233°C (Zers.)
R_{f}=0,14, I (95:5)
MS (FAB): m/z = 952 (M+H)⁺

Wie für die Beispiele 8 und 9 beschrieben, erhält man durch Kupplung der entsprechenden Säuren mit den Aminhydrochloriden (Startmaterialien) die in der Tabelle 1 aufgeführten Produkte:

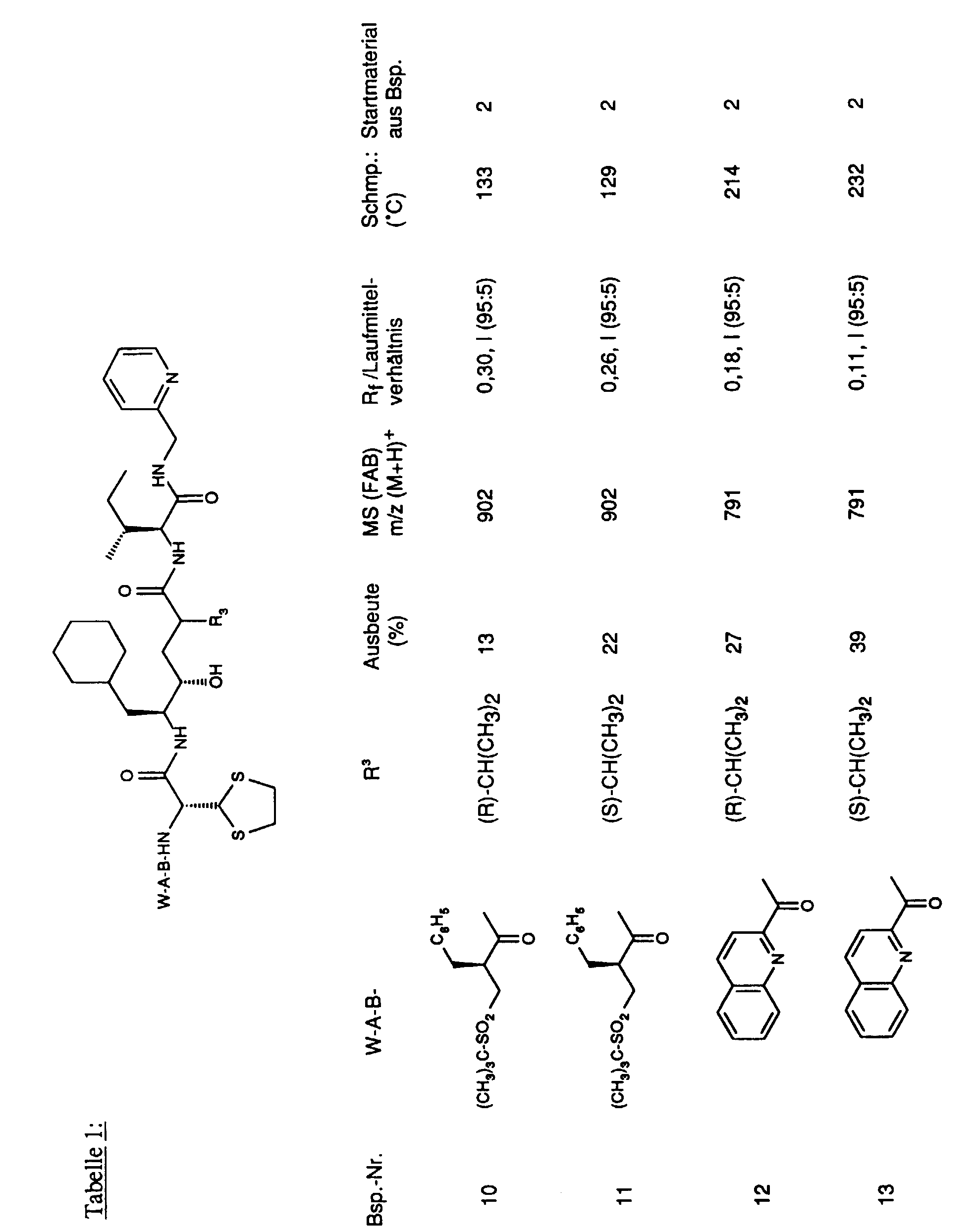

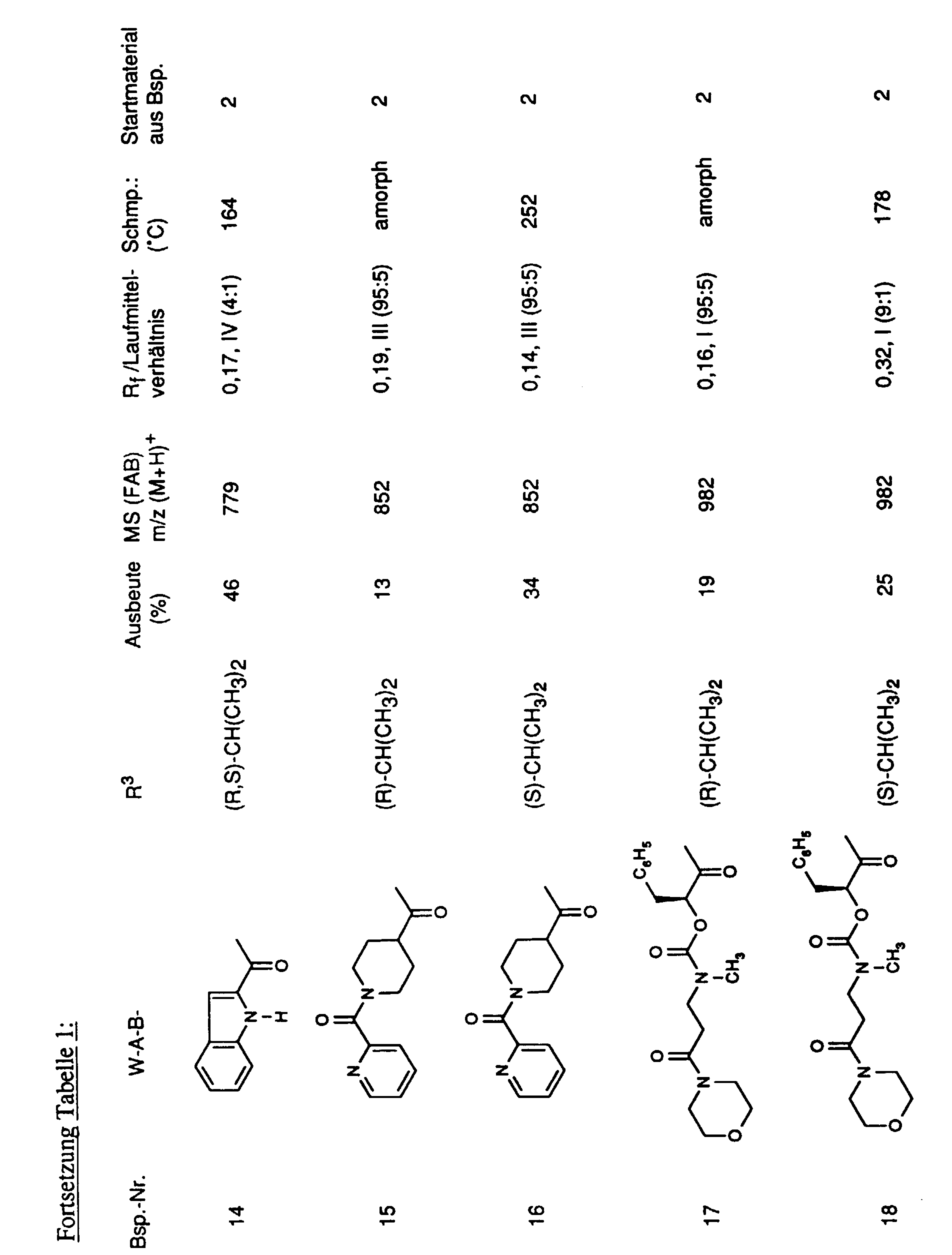

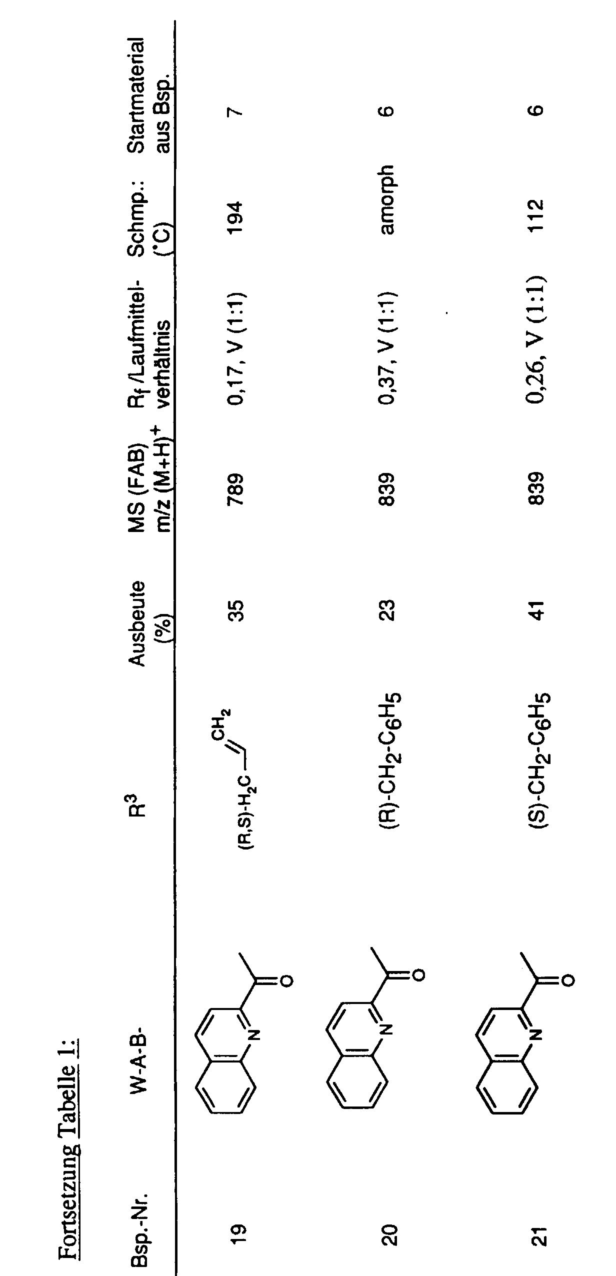

## Patentansprüche

1. Verbindungen der allgemeinen Formel (I) 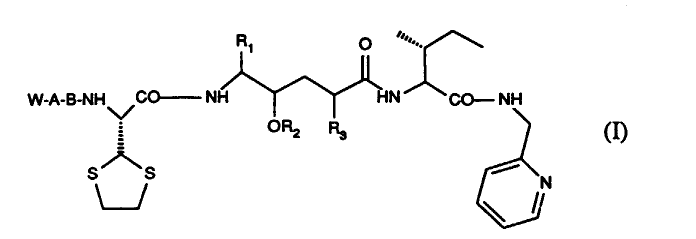 in welcher
W für Wasserstoff oder für eine typische Aminoschutzgruppe steht, oder für eine Gruppe der Formel R⁴-CO- steht,
worin
R⁴ Chinolyl, Indolyl, Pyridyl, Morpholino oder Piperazinyl bedeutet, oder
einen Rest der Formel 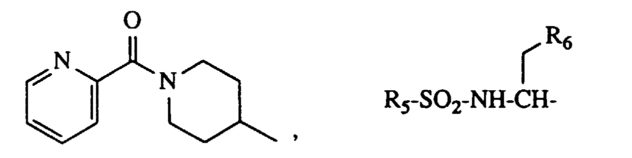 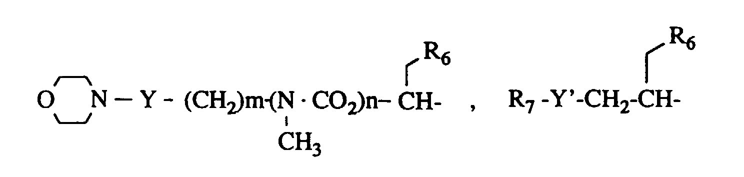 oder 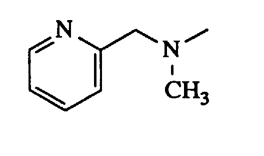 bedeutet,
worin
R⁵ geradkettiges oder verzweigtes Alkyl mit bis zu 14 Kohlenstoffatomen bedeutet, das gegebenenfalls durch Phenyl oder Naphthyl substituiert ist, oder Benzyloxy oder Aryl mit 6 bis 10 Kohlenstoffatomen bedeutet, welches seinerseits durch Alkyl mit bis zu 4 Kohlenstoffatomen substituiert ist,
oder über N-gebundenes Morpholino oder Pyrrolidinyl bedeutet,
R⁶ Phenyl oder Naphthyl bedeutet,
R⁷ die oben angegebene Bedeutung von R⁵ hat aber nicht für über N-gebundenes Morpholino oder Pyrrolidinyl steht,
Y und Y' unabhängig voneinander die CO- oder SO₂-Gruppe bedeuten,
m eine Zahl 0, 1 oder 2 bedeutet,
n eine Zahl 0 oder 1 bedeutet,
A und B gleich oder verschieden sind und für eine direkte Bindung oder
für einen Rest der Formel 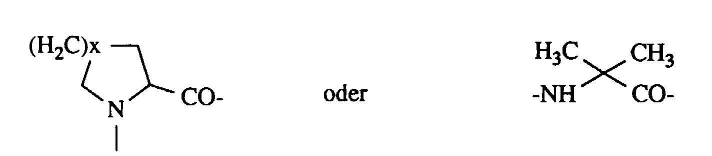 stehen ,
worin
x die Zahl 1 oder 2 bedeutet
oder
für eine Gruppe der Formel 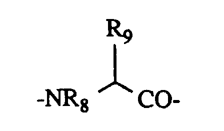 stehen
worin
R⁸ Wasserstoff oder geradkettiges oder verzweigtes Alkyl mit bis zu 4 Kohlenstoffatomen bedeutet,
R⁹ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,
wobei das Alkyl gegebenenfalls durch Cyano, Methylthio, Hydroxy, Mercapto, Guanidyl oder durch eine Gruppe der Formel -NR¹⁰R¹¹ oder R¹²-OC- substituiert ist,
worin
R¹⁰ und R¹¹ unabhängig voneinander für Wasserstoff, geradkettiges oder verzweigtes Alkyl mit bis zu 8 Kohlenstoffatomen oder Phenyl stehen,
und
R¹² Hydroxy, Benzyloxy, Alkoxy mit bis zu 6 Kohlenstoffatomen oder die oben aufgeführte Gruppe -NR¹⁰R¹¹ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe -NR¹⁰R¹¹ substituiert ist,
worin
R¹⁰ und R¹¹ die oben angegebene Bedeutung haben,
oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,
R¹ für geradkettiges oder verzweigtes Alkyl mit bis zu 3 Kohlenstoffatomen steht, das durch Cyclohexyl oder Phenyl substituiert ist,
R² für Wasserstoff oder für geradkettiges oder verzweigtes Alkyl mit bis zu 4 Kohlenstoffatomen steht, oder für eine Hydroxyschutzgruppe steht,
R³ für geradkettiges oder verzweigtes Alkyl oder Alkenyl mit jeweils bis zu 6 Kohlenstoffatomen oder für Benzyl steht,
und deren physiologisch unbedenklichen Salze.

2. Verbindungen der allgemeinen Formel (I) gemäß Anspruch 1,
in welcher
W für Wasserstoff, tert.Butyloxycarbonyl (BOC), 9-Fluorenylmethyloxycarbonyl (Fmoc), Benzyloxycarbonyl (Z) oder Pyridylmethoxycarbonyl steht, oder für eine Gruppe der Formel R⁴-CO- steht
worin
R⁴ Chinolyl, Indolyl, Pyridyl, Morpholino oder Piperazinyl bedeutet, oder
einen Rest der Formel 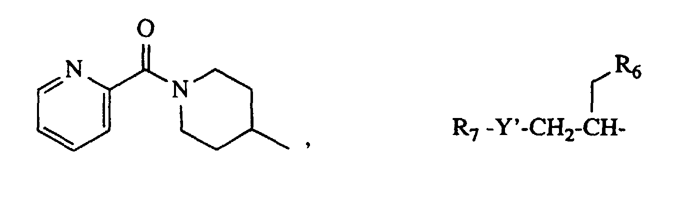 oder 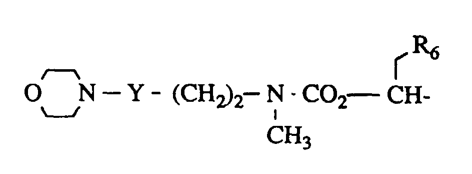 bedeutet,
worin
Y und Y' unabhänging voneinander die CO- oder SO₂-Gruppe bedeuten,
R⁶ Phenyl oder Naphthyl bedeutet,
R⁷ geradkettiges oder verzweigtes Alkyl mit bis zu 8 Kohlenstoffatomen, Tolyl, Benzyloxy, Phenyl oder Naphthyl bedeutet,
A und B unabhängig voneinander
für eine direkte Bindung oder
für Prolin, Alanin, Arginin, Asparaginsäure, Asparagin, Glutaminsäure, Glutamin, Histidin, Leucin, Methionin, Threonin, Tyrosin, Cystein, Glycin, Isoleucin, Lysin, Phenylalanin, Serin, Tryptophan oder Valin stehen,
R¹ für Benzyl oder für Methylcyclohexyl steht,
R² für Wasserstoff steht,
R³ für geradkettiges oder verzweigtes Alkyl oder Alkenyl mit jeweils bis zu 4 Kohlenstoffatomen oder Benzyl steht,
und deren physiologisch unbedenklichen Salze.

3. Verbindungen der allgemeinen Formel (I) gemäß Anspruch 1,
in welcher
W für Wasserstoff oder tert.Butyloxycarbonyl (BOC) steht, oder für eine Gruppe der Formel R⁴-CO- steht,
worin
R⁴ Chinolyl oder Indolyl bedeutet, oder
einen Rest der Formel 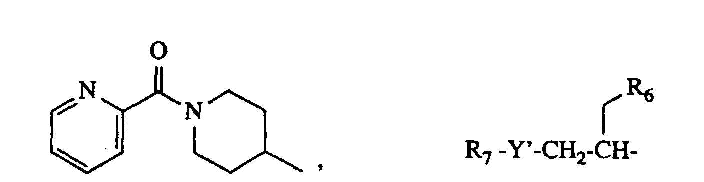 oder 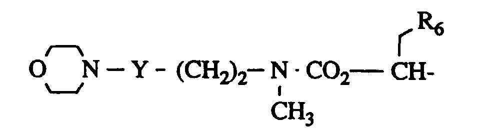 bedeutet,
worin
R⁶ Phenyl oder Naphthyl bedeutet
R⁷ geradkettiges oder verzweigtes Alkyl mit bis zu 4 Kohlenstoffatomen, Tolyl, Benzyloxy, Phenyl oder Naphthyl bedeutet,
Y und Y' unabhängig voneinander die CO- oder SO₂-Gruppe bedeuten,
A und B unabhängig voneinander
für eine direkte Bindung, oder
für Phenylalanin stehen,
R¹ für Benzyl oder Methylcyclohexyl steht,
R² für Wasserstoff steht,
R³ für geradkettiges oder verzweigtes Alkyl mit bis zu 3 Kohlenstoffatomen, Allyl oder Benzyl steht
und deren physiologisch unbedenklichen Salze.

4. Verfahren zur Herstellung von Verbindungen gemäß den Ansprüchen 1 bis 3, dadurch gekennzeichnet, daß man
im Fall, daß A und B für eine direkte Bindung stehen,
Verbindungen der allgemeinen Formel (III) 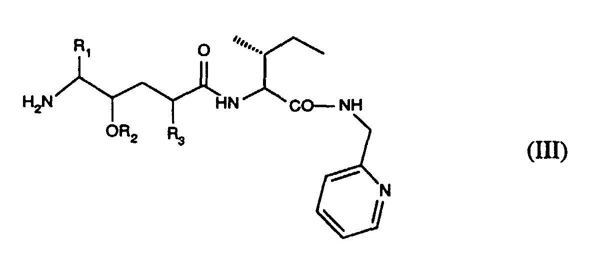 in welcher
R¹, R² und R³ die oben angegebene Bedeutung haben,
zunächst mit Verbindungen der allgemeinen Formel (IV) 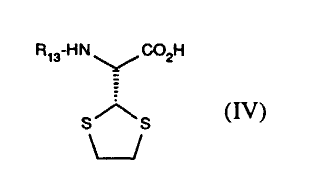 in welcher
R¹³ für eine der oben aufgeführten Aminoschutzgruppen, vorzugsweise für Boc steht,
in inerten organischen Lösemitteln, in Anwesenheit einer Base und eines Hilfsstoffes umsetzt,
anschließend die Schutzgruppe R¹³ nach denen in der Peptidchemie üblichen Methoden abspaltet (W = H) und im Fall, daß W ≠ H, in einem letzten Schritt mit Verbindungen der allgemeinen Formel (V)
W'-X (V)
in welcher
W' die oben angegebene Bedeutung von W hat, aber nicht für Wasserstoff steht,
und
X in Abhängigkeit von der jeweiligen Bedeutung der unter W oben aufgeführten Substituenten für Hydroxy oder Halogen steht,
ebenfalls in organischen Lösemitteln und in Anwesenheit einer Base und eines Hilfsstoffes umsetzt,
und gegebenenfalls eine Trennung der Diastereomere durchführt.

5. Verfahren zur Herstellung von Verbindungen gemäß den Ansprüchen 1 bis 3, dadurch gekennzeichnet, daß man im Fall, daß A und/oder B nicht für eine direkte Bindung stehen,
Verbindungen der allgemeinen Formel (III) entweder direkt mit Verbindungen der allgemeinen Formel (VI) 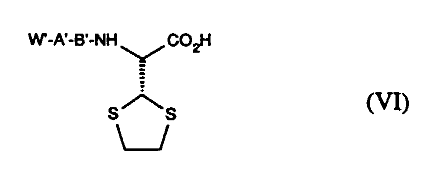 in welcher
W' die oben angegebene Bedeutung hat
und
A' und B' die oben angegebene Bedeutung von A und B haben aber nicht gleichzeitig für eine Bindung stehen,
oder sukzessive zunächst entweder mit Verbindungen der allgemeinen Formel (VII) 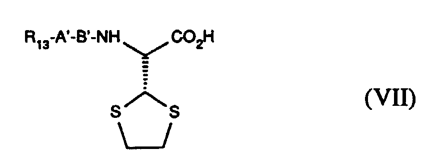 in welcher
R¹³, A' und B' die oben angegebene Bedeutung haben,
in inerten Lösemitteln und in Anwesenheit einer Base und eines Hilfsstoffes umsetzt,
anschließend unter vorgeschalteter Abspaltung der Schutzgruppe R¹³ wie im Anspruch 4 beschrieben, mit Verbindungen der allgemeinen Formel (V) umsetzt,
oder
Verbindungen der allgemeinen Formel (III) zunächst mit Verbindungen der allgemeinen Formel (IV) wie unter [A] beschrieben umsetzt, die Schutzgruppe R¹³ abspaltet und in einem letzten Schritt mit Verbindungen der allgemeinen Formel (VIII)
W'-A'-B'-OH (VIII)
in welcher
W', A' und B' die oben angegebene Bedeutung haben,
nach denen, in der Peptidchemie üblichen Methoden, gegebenenfalls unter Aktivierung der Carbonsäurefunktion und unter einer vorgeschalteten Deblockierung der Aminfunktion, in inerten organischen Lösemitteln und in Anwesenheit einer Base und eines Hilfststoffes umsetzt,
und im Fall, daß W für Wasserstoff steht, wie im Anspruch 4 beschrieben, die Schutzgruppe abspaltet,
und gegebenenfalls eine Trennung der Diastereomere durchführt.

6. Arzneimittel enthaltend eine oder mehrere Verbindungen gemäß den Ansprüchen 1 bis 3.

7. Verwendung der Verbindungen gemaß den Ansprüchen 1 bis 3 zur Herstellung von Arzneimitteln.

## Claims

1. Compounds of the general formula (I) 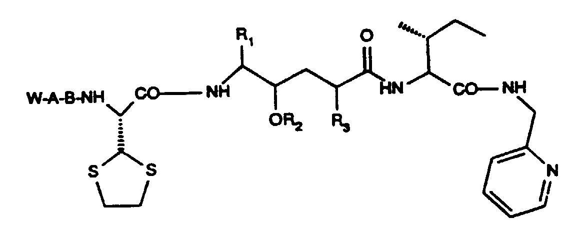 in which
W represents hydrogen or a typical amino protective group, or represents a group of the formula R⁴-CO-,
in which
R⁴ denotes quinolyl, indolyl, pyridyl, morpholino or piperazinyl, or denotes a radical of the formula 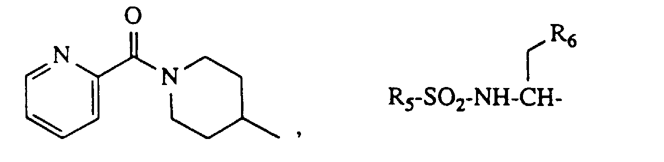 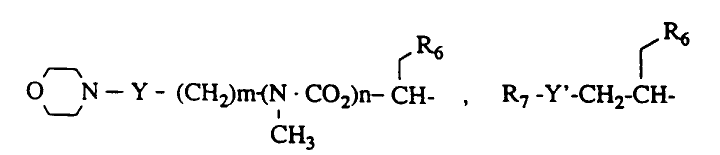 or 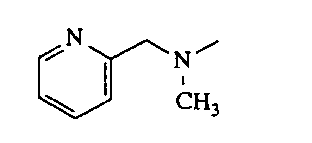 in which
R⁵ denotes straight-chain or branched alkyl having up to 14 carbon atoms, which is optionally substituted by phenyl or naphthyl, or denotes benzyloxy or aryl having 6 to 10 carbon atoms, which is in turn substituted by alkyl having up to 4 carbon atoms,
or denotes morpholino or pyrrolidinyl bonded via N,
R⁶ denotes phenyl or naphthyl,
R⁷ has the abovementioned meaning of R⁵ but does not represent morpholino or pyrrolidinyl bonded via N,
Y and Y' independently of one another denote the CO or SO₂ group,
m denotes a number 0, 1 or 2,
n denotes a number 0 or 1,
A and B are identical or different and represent a direct linkage or represent a radical of the formula 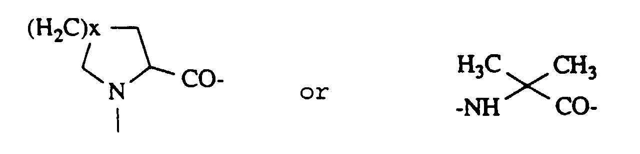 in which
x denotes the number 1 or 2
or
represent a group of the formula 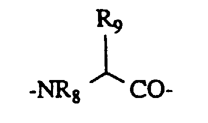 in which
R⁸ denotes hydrogen or straight-chain or branched alkyl having up to 4 carbon atoms,
R⁹ denotes cycloalkyl having 3 to 8 carbon atoms or aryl having 6 to 10 carbon atoms or hydrogen, or
denotes straight-chain or branched alkyl having up to 8 carbon atoms,
where the alkyl is optionally substituted by cyano, methylthio, hydroxyl, mercapto, guanidyl or by a group of the formula -NR¹⁰R¹¹ or R¹²-OC-,
in which
R¹⁰ and R¹¹ independently of one another represent hydrogen, straight-chain or branched alkyl having up to 8 carbon atoms or phenyl,
and
R¹² denotes hydroxyl, benzyloxy, alkoxy having up to 6 carbon atoms or the abovementioned group -NR¹⁰R¹¹,
or the alkyl is optionally substituted by cycloalkyl having 3 to 8 carbon atoms or by aryl having 6 to 10 carbon atoms, which is in turn substituted by hydroxyl, halogen, nitro, alkoxy having up to 8 carbon atoms or by the group -NR¹⁰R¹¹,
in which
R¹⁰ and R¹¹ have the abovementioned meaning,
or the alkyl is optionally substituted by a 5- to 6-membered nitrogen-containing heterocycle or indolyl, in which the corresponding -NH functions are optionally protected by alkyl having up to 6 carbon atoms or by an amino protective group,
R¹ represents straight-chain or branched alkyl having up to 3 carbon atoms, which is substituted by cyclohexyl or phenyl,
R² represents hydrogen or straight-chain or branched alkyl having up to 4 carbon atoms, or represents a hydroxyl protective group,
R³ represents straight-chain or branched alkyl or alkenyl each having up to 6 carbon atoms or represents benzyl,
and their physiologically acceptable salts.

2. Compounds of the general formula (I) according to Claim 1
in which
W represents hydrogen, tert-butoxycarbonyl (BOC), 9-fluorenylmethoxycarbonyl (Fmoc), benzyloxycarbonyl (Z) or pyridylmethoxycarbonyl,
or
represents a group of the formula R⁴-CO-
in which
R⁴ denotes quinolyl, indolyl, pyridyl, morpholino or piperazinyl, or denotes a radical of the formula 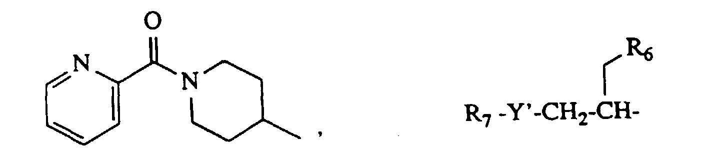 or 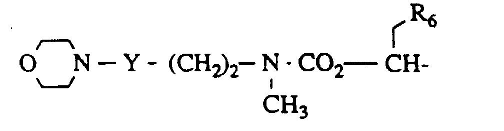 in which
Y and Y' independently of one another denote the CO or SO₂ group,
R⁶ denotes phenyl or naphthyl,
R⁷ denotes straight-chain or branched alkyl having up to 8 carbon atoms, tolyl, benzyloxy, phenyl or naphthyl,
A and B independently of one another represent a direct linkage or represent proline, alanine, arginine, aspartic acid, asparagine, glutamic acid, glutamine, histidine, leucine, methionine, threonine, tyrosine, cysteine, glycine, isoleucine, lysine, phenylalanine, serine, tryptophan or valine,
R¹ represents benzyl or methylcyclohexyl,
R² represents hydrogen,
R³ represents straight-chain or branched alkyl or alkenyl each having up to 4 carbon atoms, or benzyl,
and their physiologically acceptable salts.

3. Compounds of the general formula (I) according to Claim 1,
in which
W represents hydrogen or tert-butoxycarbonyl (BOC)
or
represents a group of the formula R⁴-CO-,
in which
R⁴ denotes quinolyl or indolyl, or denotes a radical of the formula 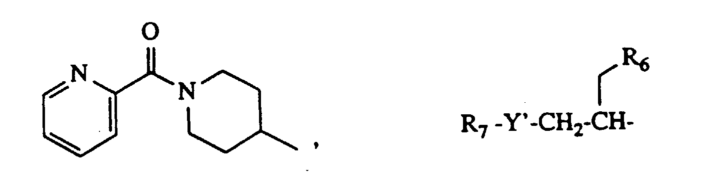 or 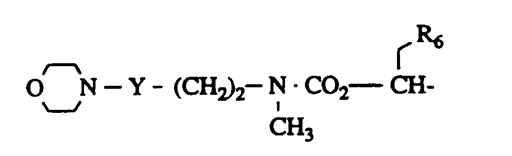 in which
R⁶ denotes phenyl or naphthyl,
R⁷ denotes straight-chain or branched alkyl having up to 4 carbon atoms, tolyl, benzyloxy, phenyl or naphthyl,
Y and Y' independently of one another denote the CO or
SO₂ group,
A and B independently of one another represent a direct linkage, or represent phenylalanine,
R¹ represents benzyl or methylcyclohexyl,
R² represents hydrogen,
R³ represents straight-chain or branched alkyl having up to 3 carbon atoms, allyl or benzyl,
and their physiologically acceptable salts.

4. Process for the preparation of compounds according to Claims 1 to 3, characterised in that
in the case in which A and B represent a direct linkage
compounds of the general formula (III) 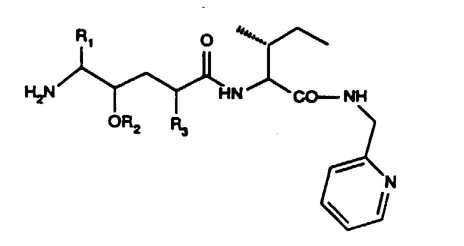 in which
R¹, R² and R³ have the abovementioned meaning,
are first reacted with compounds of the general formula (IV) 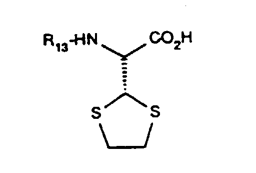 in which
R¹³ represents one of the abovementioned amino protective groups, preferably Boc,
in inert organic solvents, in the presence of a base and of an auxiliary,
then the protective group R¹³ is removed by the methods customary in peptide chemistry (W = H) and, in the case in which W ≠ H, the product is reacted in a last step with compounds of the general formula (V)
W'-X (V)
in which
W' has the abovementioned meaning of W, but does not represent hydrogen
and
X, depending on the particular meaning of the substituents mentioned above under W, represents hydroxyl or halogen,
likewise in organic solvents and in the presence of a base and of an auxiliary,
and, if appropriate, a separation of the diastereomers is carried out.

5. Process for the preparation of compounds according to Claims 1 to 3, characterised in that, in the case in which A and/or B does not represent a direct linkage,
compounds of the general formula (III) are either reacted directly with compounds of the general formula (VI) 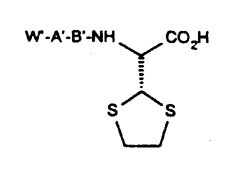 in which
W' has the abovementioned meaning
and
A' and B' have the abovementioned meaning of A and B but do not simultaneously represent a bond,
or are reacted successively first either with compounds of the general formula (VII) 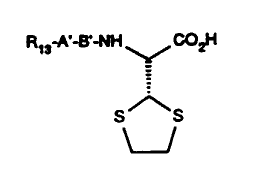 in which
R¹³, A' and B' have the abovementioned meaning,
in inert solvents and in the presence of a base and of an auxiliary,
then, with prior removal of the protective group R¹³ as described in Claim 4, reacted with compounds of the general formula (V),
or
compounds of the general formula (III) are first reacted with compounds of the general formula (IV) as described under [A], the protective group R¹³ is removed, and in a last step reacted with compounds of the general formula (VIII)
W'-A'-B'-OH (VIII)
in which
W', A' and B' have the abovementioned meaning,
by the methods customary in peptide chemistry, it appropriate with activation of the carboxylic acid function and with prior deblocking of the amine function, in inert organic solvents and in the presence of a base and of an auxiliary,
and in the case in which W represents hydrogen, the protective group is removed as described in Claim 4,
and, if appropriate, a separation of the diastereomers is carried out.

6. Medicaments containing one or more compounds according to Claims 1 to 3.

7. Use of the compounds according to Claims 1 to 3 for the production of medicaments.

## Revendications

1. Composés de formule générale (I) 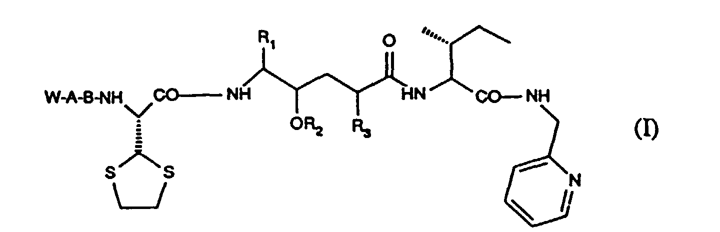 dans laquelle
W représente de l'hydrogène ou un groupe classique protégeant la fonction amino, ou un groupe de formule R⁴-CO-,
dans laquelle
R⁴ est un groupe quinolyle, indolyle, pyridyle, morpholino ou pipérazinyle, ou bien un reste de formule 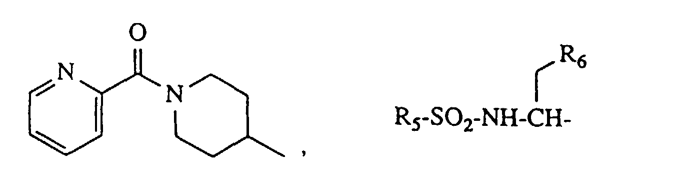 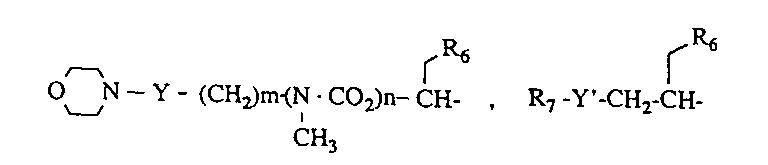 ou 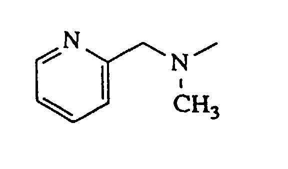 dans laquelle
R⁵ est un groupe alkyle linéaire ou ramifié ayant jusqu'à 14 atomes de carbone, qui est éventuellement substitué par un reste phényle ou naphtyle, ou un groupe benzyloxy ou aryle ayant 6 à 10 atomes de carbone, qui est substitué de son côté par un reste alkyle ayant jusqu'à 4 atomes de carbone,
ou désigne un groupe morpholino ou pyrrolidinyle lié par l'azote,
R⁶ est un groupe phényle ou naphtyle,
R⁷ a la définition indiquée ci-dessus pour R⁵, mais ne représente pas un groupement morpholino ou pyrrolidinyle lié par l'azote,
Y et Y' représentent, indépendamment l'un de l'autre, le groupe CO ou SO₂,
m est le nombre 0, 1 ou 2,
n est le nombre 0 ou 1,
A et B sont identiques ou différents et représentent une liaison directe ou un reste de formule 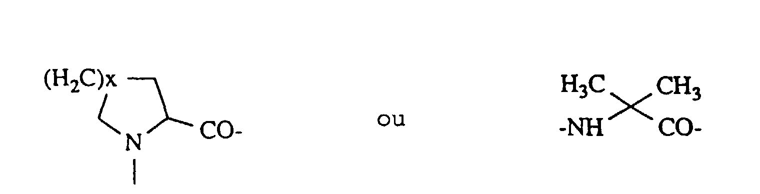 dans laquelle
x représente le nombre 1 ou 2
ou bien
un groupe de formule 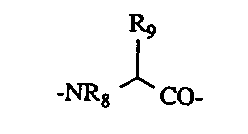 dans laquelle
R⁸ est de l'hydrogène ou un groupe alkyle linéaire ou ramifié ayant jusqu'à 4 atomes de carbone,
R⁹ est un groupe cycloalkyle ayant 3 à 8 atomes de carbone ou un groupe aryle ayant 6 à 10 atomes de carbone ou de l'hydrogène, ou bien
un groupe alkyle linéaire ou ramifié ayant jusqu'à 8 atomes de carbone,
le groupe alkyle étant éventuellement substitué par un reste cyano, méthylthio, hydroxy, mercapto, guanidyle ou par un groupe de formule -NR¹⁰R¹¹ ou R¹²-OC-,
dans laquelle
R¹⁰ et R¹¹ représentent, indépendamment l'un de l'autre, de l'hydrogène, un groupe alkyle linéaire ou ramifié ayant jusqu'à 8 atomes de carbone ou un groupe phényle,
et
R¹² est un groupe hydroxy, benzyloxy, alkoxy ayant jusqu'à 6 atomes de carbone ou représente le groupe -NR¹⁰R¹¹ indiqué ci-dessus, ou bien le groupe alkyle est substitué, le cas échéant, par un reste cycloalkyle ayant 3 à 8 atomes de carbone ou par un reste aryle ayant 6 à 10 atomes de carobne, qui est substitué de son côté par un reste hydroxy, halogéno, nitro, alkoxy ayant jusqu'à 8 atomes de carbone ou par le groupe -NR¹⁰R¹¹,
dans lequel
R¹⁰ et R¹¹ ont la définition indiquée ci-desssus,
ou bien le groupe alkyle est substitué par un hétérocycle pentagonal ou hexagonal contenant de l'azote ou par un groupe indolyle, les fonctions -NH correspondantes étant protégées, le cas échéant, par un groupe alkyle ayant jusqu'à 6 atomes de carbone ou par un groupe protégeant la fonction amino,
R¹ est un groupe alkyle linéaire ou ramifié ayant jusqu'à 3 atomes de carbone, qui est substitué par un reste cyclohexyle ou phényle,
R² représente de l'hydrogène ou un groupe alkyle linéaire ou ramifié ayant jusqu'à 4 atomes de carbone, ou représente un groupe protégeant la fonction hydroxy,
R³ est un groupe alkyle ou un groupe alcényle linéaire ou ramifié ayant chacun jusqu'à 6 atomes de carbone ou un groupe benzyle,
et leurs sels acceptables du point de vue physiologique.

2. Composés de formule générale (I) suivant la revendication 1,
dans laquelle
W représente de l'hydrogène, un groupe tertiobutyloxycarbonyle (BOC), 9-fluorénylméthyloxycarbonyle (Fmoc), benzyloxycarbonyle (Z) ou pyridylméthoxycarbonyle, ou bien un groupe de formule R⁴-CO-
dans laquelle
R⁴ est un groupe quinolyle, indolyle, pyridyle, morpholino ou pipérazinyle, ou bien un reste de formule 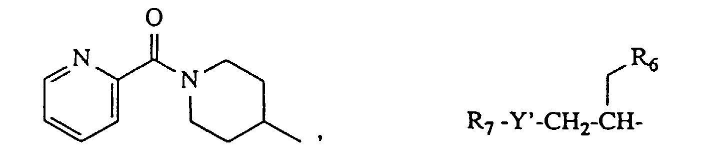 ou 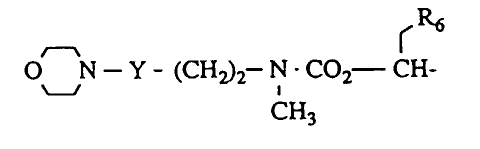 dans laquelle
Y et Y' représentent, indépendamment l'un de l'autre, un groupe CO ou SO₂,
R⁶ est un groupe phényle ou naphtyle,
R⁷ est un groupe alkyle linéaire ou ramifié ayant jusqu'à 8 atomes de carbone, un groupe tolyle, benzyloxy, phényle ou naphtyle,
A et B représentent, indépendamment l'un de l'autre, une liaison directe ou bien
la proline, l'alanine, l'arginine, l'acide aspartique, l'asparagine, l'acide glutamique, la glutamine, l'histidine, la leucine, la méthionine, la thréonine, la tyrosine, la cystéine, la glycine, l'isoleucine, la lysine, la phénylalanine, la sérine, le tryptophane ou la valine,
R¹ est un groupe benzyle ou un groupe méthylcyclohexyle,
R² est de l'hydrogène,
R³ est un groupe alkyle ou un groupe alcényle linéaire ou ramifié ayant chacun jusqu'à 4 atomes de carbone ou un groupe benzyle,
et leurs sels acceptables du point de vue physiologique.

3. Composés de formule générale (I) suivant la revendication 1,
dans laquelle
W est de l'hydrogène ou un groupe tertio-butyloxycarbonyle (BOC), ou bien un groupe de formule R⁴-CO-,
dans laquelle
R⁴ est un reste quinolyle ou indolyle, ou bien un reste de formule 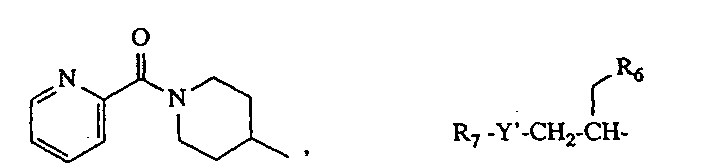 ou 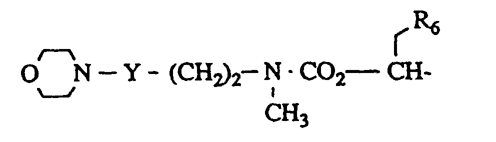 dans laquelle
R⁶ est un groupe phényle ou naphtyle,
R⁷ est un groupe alkyle linéaire ou ramifié ayant jusqu'à 4 atomes de carbone, un groupe tolyle, benzyloxy, phényle ou naphtyle,
Y et Y' représentent, indépendamment l'un de l'autre, le groupe CO ou SO₂,
A et B représentent, indépendamment l'un de l'autre, une liaison directe ou la phénylalanine,
R¹ est un groupe benzyle ou méthylcyclohexyle,
R² est de l'hydrogène,
R³ est un groupe alkyle linéaire ou ramifié ayant jusqu'à 3 atomes de carbone, un groupe allyle ou benzyle,
et leurs sels acceptables du point de vue physiologique.

4. Procédé de production de composés suivant les revendications 1 à 3, caractérisé en ce que
au cas où A et B représentent une liaison directe,
on fait réagir des composés de formule générale (III) 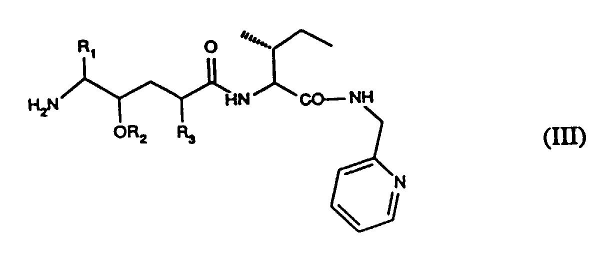 dans laquelle
R¹, R² et R³ ont la définition indiquée ci-dessus, tout d'abord avec des composés de formule générale (IV) 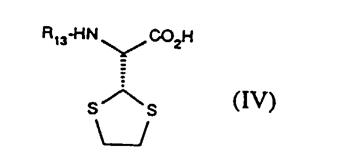 dans laquelle
R¹³ représente l'un des groupes protégeant la fonction amino indiqués ci-dessus, de préférence le groupe BOC,
dans des solvants organiques inertes, en présence d'une base et d'une substance auxiliaire,
puis on élimine le groupe protecteur R¹³ par les moyens d'emploi classiques dans la chimie des peptides (W=H) et, au cas où W est différent de H, on conduit une réaction dans une dernière étape avec des composés de formule générale (V)
W'-X (V)
dans laquelle
W' a la définition indiquée ci-dessus pour W, mais ne représente pas de l'hydrogène,
et
X représente un groupe hydroxy ou un halogène en fonction de la définition respective des substituants indiqués ci-dessus à propos de W,
également dans des solvants organiques et en présence d'une base et d'une substance auxiliaire,
et on effectue, le cas échéant, une séparation des diastéréoisomères.

5. Procédé de production de composés suivant les revendications 1 à 3, caractérisé en ce que, au cas où A et/ou B ne représentent pas une liaison directe,
on fait réagir des composés de formule générale (III) soit directement avec des composés de formule générale (VI) 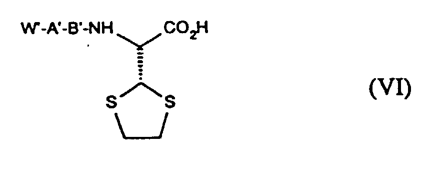 dans laquelle
W' a la définition indiquée ci-dessus
et
A' et B' ont la définition indiquée ci-dessus pour A et B, mais ne représentent pas en même temps une liaison,
ou bien successivement, tout d'abord avec des composés de formule générale (VII) 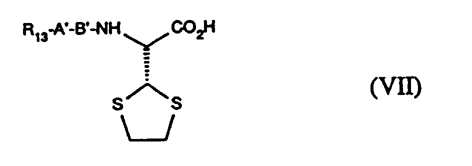 dans laquelle
R¹³, A' et B' ont la définition indiquée ci-dessus,
dans des solvants inertes et en présence d'une base et d'une substance auxiliaire,
puis après élimination préalable du groupe protecteur R¹³ comme décrit dans la revendication 4, on conduit la réaction avec des composés de formule générale (V),
ou bien
on fait réagir des composés de formule générale (III) tout d'abord avec des composés de formule générale (IV) comme décrit en [A], on élimine le groupe protecteur R¹³ et, dans une dernière étape, on conduit une réaction avec des composés de formule générale (VIII)
W'-A'-B'-OH (VIII)
dans laquelle
W', A' et B' ont la définition indiquée ci-dessus,
par les modes opératoires d'emploi classiques dans la chimie des peptides, éventuellement avec activation de la fonction acide carboxylique et avec élimination préalable de la protection de la fonction amino, dans des solvants organiques inertes et en présence d'une base et d'une substance auxiliaire,
et au cas où W représente de l'hydrogène, comme décrit dans la revendication 4, on élimine le groupe protecteur,
et on effectue, le cas échéant, une séparation des diastéréoisomères.

6. Médicament contenant un ou plusieurs composés suivant les revendications 1 à 3.

7. Utilisation des composés suivant les revendications 1 à 3 pour la préparation de médicaments.
